Europäisches Patentamt

European Patent Office

Office européen des brevets

(11) Publication number: **0 354 989 B1**

# EUROPEAN PATENT SPECIFICATION

(45) Date of publication of patent specification: **13.09.95**

(21) Application number: **89111714.5**

(22) Date of filing: **27.06.89**

(51) Int. Cl.⁶: **C12N  15/27**, C12P 21/02,
C12N 5/00, C07K 14/53,
A61K 38/22, A61K 39/395,
C12Q 1/68, G01N 33/68

(54) Megakaryocytopoietin, preparation and use.

(30) Priority: **28.06.88 US 212623**

(43) Date of publication of application:
**21.02.90 Bulletin  90/08**

(45) Publication of the grant of the patent:
**13.09.95 Bulletin  95/37**

(84) Designated Contracting States:
**AT BE CH DE ES FR GB GR IT LI LU NL SE**

(56) References cited:
**EP-A- 0 260 918**

**PROCEEDINGS OF THE NATIONAL ACADEMY
OF SCIENCE, USA, vol. 84, June 1987, pages
3555-3559, Washington, DC, US; C.A. PRODY
et al.: "Isolation and characterization of full-
length cDNA clones coding for
cholinesterase from fetal human tissues"**

(73) Proprietor: **YISSUM RESEARCH DEVELOP-
MENT COMPANY OF THE HEBREW UNIVER-
SITY OF JERUSALEM
46 Jabotinsky Street
Jerusalem, 92 182 (IL)**

(72) Inventor: **Hermona, Soreq
Rishon
Lezion (IL)**
Inventor: **Haim Zakut
Savion
Savion (IL)**

(74) Representative: **Huber, Bernhard, Dipl.-Chem.
et al
Patentanwälte
H. Weickmann, Dr. K. Fincke
F.A. Weickmann, B. Huber
Dr. H. Liska, Dr. J. Prechtel, Dr. B.
Böhm
Postfach 86 08 20
D-81635 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF CLINICAL INVESTIGATION, vol. 75, April 1985, pages 1174-1182, The American Society for Clinical Investigation, Inc., New York, US; R. HOFFMAN et al.: "Purification and partial characterization of a megakaryocyte colony-stimulating factor from human plasma"

CLINICAL RESEARCH, vol. 31, no. 4, 1983, page 769a, Thorofare, US; N.M. FARBER et al.: "Translation of mRNA from human kidneys into biologically active frythropoietin following microinjection into xenopus laevis oocytes"

**Description**

This application relates to growth factors. In particular, it relates to polypeptides which influence the replication or differentiation of blood cells, especially platelet progenitor cells.

In the bone marrow a pluripotent cell differentiates into megakaryocytic, erythrocytic, and myelocytic cell lines. There may be a line of committed cells between stem cells and megakaryocytes. The earliest recognizable member of the megakaryocyte (meg) series, the megakaryoblast, is initially 20 to 30 $\mu$m in diameter with basophilic cytoplasm and a slightly irregular nucleus having loose, somewhat reticular chromatin and several nucleoli. Later megakaryoblasts may contain up to 32 nuclei, but the cytoplasm remains sparse and immature. As maturation proceeds, the nucleus becomes more lobulated and pyknotic; the cytoplasm increases in quantity and becomes more acidophilic and granular. The most mature cells may give the appearance of releasing platelets at their periphery. Normally, less than 10% of megakaryocytes are in the blast stage and more than 50% are mature. Arbitrary morphologic classifications commonly applied to the megakaryocyte series are megakaryoblast for the earliest form; promegakaryocyte or basophilic megakaryocyte for the intermediate form; and mature (acidophilic, granular, or platelet-producing) megakaryocyte for the late forms. The mature megakaryocyte extends filaments of cytoplasm into sinusoidal spaces where they detach and fragment into individual platelets (Williams et al., Hematology, 1972).

Evidence is accumulating that there is a dual level of regulation of megakaryocytopoiesis, involving more than one regulatory factor (Williams et al., Br. J. Haematol. 52:173 [1982] and Williams et al., J. Cell Physiol. 110:101 [1982]). The early level of megakaryocytopoiesis is postulated as being mitotic, concerned with cell proliferation and colony initiation from CFU-meg but is not affected by platelet count (Burstein et al., J. Cell Physiol. 109:333 [1981] and Kimura et al., Exp. Hematol. 13:1048 [1985]). The later stage of maturation is non-mitotic, involved with nuclear polyploidization and cytoplasmic maturation and is probably regulated in a feedback mechanism by peripheral platelet number (Odell et al., Blood 48:765 [1976] and Ebbe et al., Blood 32:787 [1968]). The existence of a distinct and specific megakaryocyte colony-stimulating factor (meg-CSF) is still in dispute (Mazur, E., Exp. Hematol. 15:340-350 [1987]). Although meg-CSF's have been partly purified from experimentally produced thrombocytopenia (Hill et al., Exp. Hematol. 14:752 [1986]) and human embryonic kidney conditioned medium [CM] (McDonald et al., J. Lab. Clin. Med. 85:59 [1975]) and in man from aplastic anemia and idiopathic thrombocytopenic purpura urinary extracts (Kawakita et al., Blood 6:556 [1983]) and plasma (Hoffman et al., J. Clin. Invest. 75:1174 [1985]), their physiological function is as yet unknown in most cases. The conditioned medium of pokeweed mitogen-activated spleen cells (PWM-SpCM) and WEHI-3 (WEHI-3CM) have been used as megakaryocyte potentiators. PWM-SpCM contains factors enhancing CFU-meg growth (Metcalf et al.. PNAS; USA 72:1744-1748 [1975]; Quesenberry et al., Blood 65:214 [1985]; and Iscove, N.N., in Hematopoietic Cell Differentiation, ICN-UCLA Symposia on Molecular and Cellular Biology, Vol. 10, Golde et al., eds. [New York, Academy Press] pp 37-52 [1978]), one of which is interleukin-3 (IL-3), a multilineage colony stimulating factor (multi-CSF [Burstein, S.A., Blood Cells 11:469, 1986]). The other factors in this medium have not yet been identified and isolated. WEHI-3 is a murine myelomonocytic cell line secreting relatively large amounts of IL-3 and smaller amounts of GM-CSF. IL-3 has been recently purified and cloned (Ihle et al., J. Immunol. 129:2431 [1982]) and has been found to potentiate the growth of a wide range of hemopoietic cells (Ihle et al., J. Immunol. 13:282 [1983]). Besides this it can also synergize with many of the known hemopoietic hormones or growth factors (Bartelmez et al., J. Cell Physiol. 122:362-369 [1985] and Warren et al., Cell 46:667-674 [1988]). IL-3 has been found to synergize with both erythropoietin (EPO) and H-1 (later known as interleukin-1 or IL-1) in the induction of very early multipotential precursors and the formation of very large mixed hemopoietic colonies.

Other sources of megakaryocyte potentiators have been found in the conditioned media of murine lung, bone, macrophage cell lines, peritoneal exudate cells and human embryonic kidney cells. Despite certain conflicting data (Mazur, E., Exp. Hematol. 15:340-350 [1987]), there is some evidence (Geissler et al., Br. J. Haematol. 60:233-238 [1985]) that activated T lymphocytes rather than monocytes play an enhancing role in megakaryocytopoiesis. This may be suggestive of the idea that activated T-lymphocyte secretions such as interleukins may be regulatory factors in meg development (Geissler et al., Exp. Hematol. 15:845-853 [1987]). A number of studies on megakaryocytopoiesis with purified EPO (Vainchenker et al., Blood 54:940 [1979]; McLeod et al., Nature 261:492-4 [1976]; and Williams et al., Exp. Hematol. 12:734 [1984]) indicate that this hormone has an enhancing effect on meg colony formation. More recently this has been demonstrated in both serum-free and serum-containing cultures and in the absence of accessory cells (Williams et al., Exp. Hematol. 12:734 [1984]). EPO was postulated to be involved more in the single and two-cell stage aspects of megakaryocytopoiesis as opposed to the effect of PWM-SpCM which was

EP 0 354 989 B1

involved in the four-cell stage of megakaryocyte development.

EP-A-0 260 918 discloses a megakaryocyte stimulatory factor isolated from human kidney cells and having a molecular weight of 15,000 on SDS-PAGE and an isoelectric point of 5.1. In Hoffmann et al. (J. Clin. Invest. 75 (1985), 1174-1182) a megakaryocyte colony-stimulating factor obtained from human plasma and having a molecular weight of 46,000 on SDS-PAGE is described.

The interaction of all these factors on both early and late phases of megakaryocyte development remains to be elucidated.

Platelets are critical formed elements of the blood clotting mechanism. Depletion of the circulating level of platelets, called thrombocytopenia, occurs in various clinical conditions and disorders. Increased rates of platelet destruction characterize immunologic or nonimmunologic acquired thrombocytopenia such as that which is associated with infections, Moschcowitz' Syndrome, Gasser's Syndrome, direct or drug-induced platelet toxicity, post-transfusion purpura and allergic thrombocytopenia; idiopathic thrombocytopenic purpura; and immunologic or nonimmunologic congenital thrombocytopenia such as erythroblastosis fetalis, prematurity thrombocytopenia, infectious thrombocytopenia, thrombocytopenia with giant cavernous hemangioma, drug sensitivity, isoimmune neonatal thrombocytopenia, and thrombocytopenia associated with maternal idiopathic thrombocytopenic purpura. Diminished or defective platelet production characterizes Fanconi Syndrome, amegakaryocytic thrombocytopenia, hereditary thrombocytopenia, neonatal rubella, maternal ingestion of thiazide diuretics, aplastic anemia, malignant infiltration of marrow, and the administration of ionizing radiation and myelosuppressive drugs.

Thrombocytopenia is clinically dangerous, exposing patients with this condition to uncontrolled bleeding episodes. In the ordinary course of events, the time required for maturation of platelets from megakaryocytes after an insult is about 4 to 5 days in humans. It would be desirable to identify an agent capable of accelerating platelet development and shortening this period.

Considerable efforts have been expended on identifying such an agent, commonly referred to as thrombopoeitin. The activity of such an agent has been observed as early as 1959 (Rak et al., "Med. Exp." 1:125) and attempts to characterize and purify this agent have continued up to the present day. Some have reported partial purification of thrombopoeitin-active polypeptides (see, for example, Tayrien et al., "J. Biol. Chem." 262:3262, 1987; and Hoffman et al., "J. Clin. Invest." 75:1174, 1985) while others have postulated that thrombopoeitin is not a discrete entity in its own right but rather is simply the polyfunctional manifestation of a known hormone (IL-3, Sparrow et al., "Prog. Clin. Biol. Res." 215 (Megakaryocyte Dev. Funct.):123, 1986).

Accordingly, it is an object of this invention to obtain a thrombopoeitin-active polypeptide which is in a pharmaceutically pure form.

It is another object to provide nucleic acid encoding the polypeptide and to use this nucleic acid to produce the polypeptide in recombinant cell culture for diagnostic use or for therapeutic use in the treatment of thrombocytopenia.

It is still further an object to synthesize variants of the polypeptide, including amino acid sequence and covalent derivatives thereof.

Another object is to produce antibodies capable of binding to the polypeptide.

A still further object is to provide diagnostic methods for the determination of the polypeptide, or nucleic acid or its antibodies, in test samples which methods are useful in the detection and classification of leukemias.

These and other objects of the invention will be apparent to the ordinary artisan upon consideration of the specification as a whole.

Summary of the Invention

The subject-matter of the present invention is as claimed in claims 1 to 13.

The present invention relates to the identification of a gene encoding a thrombopoietin-active polypeptide (hereinafter, megakaryocytopoeitin or "MKP"). A DNA sequence encoding a butyryl-cholinesterase derivative was fortuitously discovered during an investigation of multiple cDNA libraries from human fetal tissues with butyryl-cholinesterase cDNA probes. This derivative contained the first exon from the human butyryl-cholinesterase gene (250 nucleotides of the 5' region of the butyryl-cholinesterase coding sequence). However, the 3' end of this clone (hereinafter "clone 14") contained an open reading frame encoding a polypeptide other than the remainder of butyryl-cholinesterase. The DNA at the 3' end of the butyryl-cholinesterase derivative was further found to hybridize to genomic DNA fragments obtained in an acute myelocytic leukemia.

4

Nucleic acid comprising the clone 14 sequence is provided in order to prepare MKP by recombinant culture. Nucleic acid which is hybridizable to the clone 14 sequence under stringent conditions (but which may not encode the clone 14 polypeptide or any portion thereof) is used to probe for sources of DNA encoding related polypeptides or for tissues or cell lines which are transcribing this gene or for primer extension.

Also within the scope of this invention are therapeutic preparations of MKP obtained by conventional techniques from native or recombinant sources of MKP, antibodies against MKP and diagnostic methods for the determination of MKP, its nucleic acid or its antibodies.

Brief Summary of the Drawing

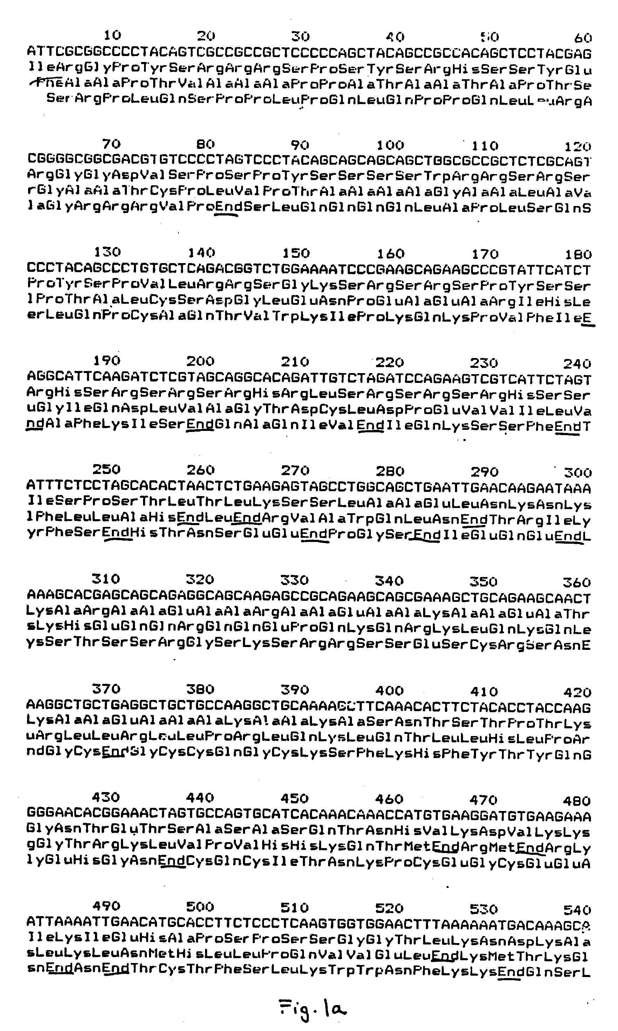

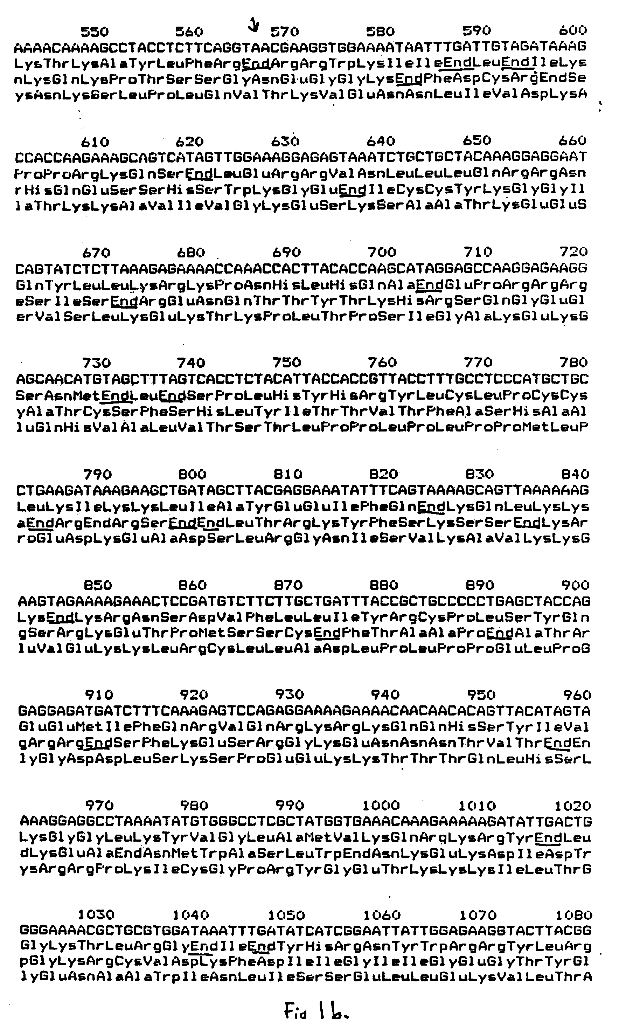

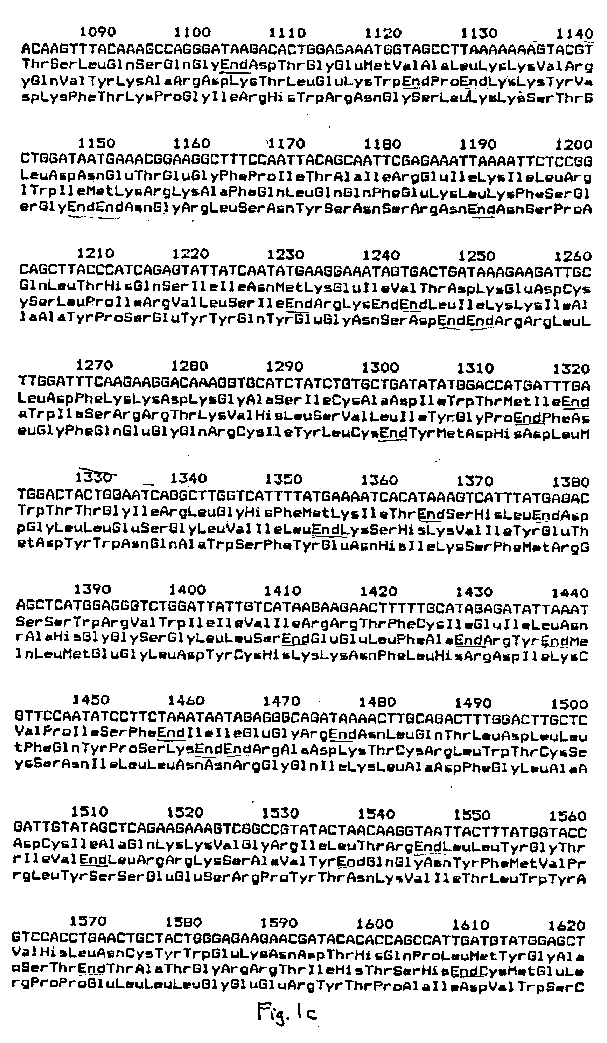

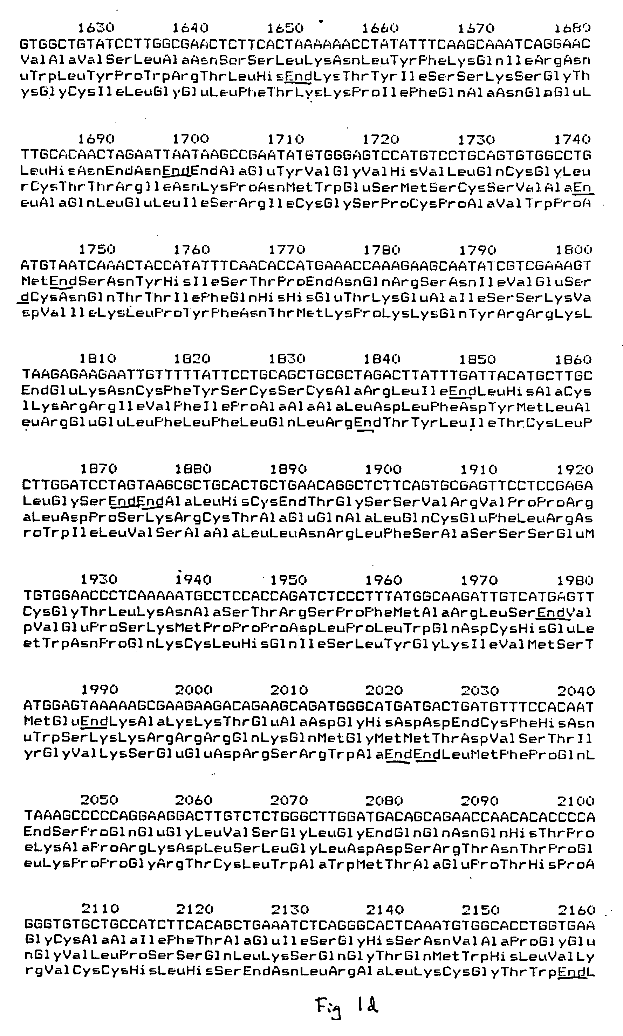

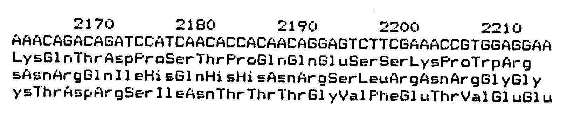

Figs. 1a - 1e depict the nucleotide and imputed amino acid sequences of clone 14. The upper reading frame encoded by nucleotides 1 to 564 imputes a polypeptide found within full-length MKP, probably representing the C-terminus of MKP, and is referred to herein as the sequence or polypeptide of Fig. 1..

Detailed Description of the Invention

MKP is a hormonally-active polypeptide believed to have utility in the modulation of hematopoiesis and immune responses, and in diagnostic testing. MKP, its immunogens, its antibodies and nucleic acid which hybridizes to nucleic acid encoding MKP are diagnostically useful in the subclassification of rare leukemias. MKP polypeptide is employed in standards for the detection of MKP expression by leukemias. Nucleic acid which is capable of hybridizing to MKP-encoding RNA finds utility in the detection of MKP gene amplification in those same leukemias. MKP immunogens are useful for the manufacture of antibodies to MKP, which in turn are useful in immunoassays for MKP as will be more fully described below.

The hematopoietic and immunomodulatory characteristics of MKP are resolved by conventional screening assays which measure the influence of MKP on the growth and/or differentiation of blood cells and their progenitors, including erythrocytes, megakaryocytes, stem cells, monocytes, lymphocytes and the like. Based on preliminary data, it is believed that MKP potentiates the development or differentiation of megakaryocytes and has utility in therapeutic conditions where such an activity is required. However, it will be understood that MKP is likely to be polyfunctional and the determination of other activities, and hence uses, for MKP will be within the ordinary skill of the artisan.

MKP is defined as a polypeptide having the sequence set forth in the designated upper reading frame of Fig. 1 together with amino acid sequence variants thereof wherein one or more amino acid residues have been inserted, deleted or substituted and wherein the variants exhibit substantially the same qualitative biological activity as the polypeptide of Fig. 1 or its full-length, mature counterpart.

The biological activity of MKP is defined as the ability to modulate the growth or differentiation of blood cells or their progenitor cells, or the ability to cross-react with an antibody raised against the polypeptide of Fig. 1. Preferably, the biological activity of MKP is determined by the modified Iscove's method described in the example below, or by other conventional assays known to those in the field.

MKP variants will have sequences that ordinarily are greater than about 80 percent homologous with the designated sequence of Fig. 1, having aligned the sequences to achieve maximum homology and not considering as homologous any conservative substitutions. N- or C-terminal substitutions shall not be construed as reducing the homology of the variant to the Fig. 1 sequence. MKP variants exclude any polypeptide heretofore identified. Typical variants of the Fig. 1 sequence found in nature may include the full-length analogue of MKP, counterparts from animals such as porcine, non-human primate, equine, murine and ovine, and alleles of the foregoing. Other amino acid sequence variants are predetermined and prepared by site-directed mutagenesis.

Amino acid sequence variants of MKP include predetermined deletions from, or insertions or substitutions of residues within the MKP sequence shown in Fig. 1 or its mature homologue. Amino acid sequence deletions generally range from about 1 to 10 residues and typically are contiguous. Contiguous deletions ordinarily are made in even numbers of residues, but single or odd numbers of deletions are within the scope hereof.

Insertions also are preferably made in even numbers of residues although insertions may range from 1 to 5 residues in general. However, insertions also include fusions onto one or both of the amino or carboxyl termini of MKP, ranging in length from 1 residue to polypeptides containing one hundred or more residues. An example of a single terminal insertion is mature MKP having an N-terminal methionyl. This variant is an artifact of the direct expression of MKP in recombinant cell culture, i.e., expression without a signal sequence to direct the secretion of mature MKP. Other examples of terminal insertions include 1) fusions of heterologous signal sequences to the N-terminus of mature MKP in order to facilitate the secretion of mature MKP from recombinant hosts, 2) fusions of immunogenic polypeptides, e.g. bacterial polypeptides such as beta-lactamase or an enzyme encoded by the E. coli trp locus and 3) N-terminal fusions with cholinesterases or N-terminal fragments of cholinesterases, especially butyryl-cholinesterase. The antibody and MKP are covalently bonded, for example, by the method of EP 170,697A, although other methods for linking proteins are conventional and known to the artisan. Immunogenic fusions are useful for preparing immunogenic MKPs suitable as vaccines for preparing anti-MKP antibodies.

The third group of variants are those in which at least one residue in the MKP molecule has been removed and a different residue inserted in its place. Such substitutions generally are made in accordance with following Table.

Table 1

| Original Residue | Exemplary Substitutions |
| --- | --- |
| Ala | gly; ser |
| Arg | lys |
| Asn | gln; his |
| Asp | glu |
| Cys | ser |
| Gln | asn |
| Glu | asp |
| Gly | ala |
| His | asn; gln |
| Ile | leu; val |
| Leu | ile; val |
| Lys | arg; gln; glu |
| Met | met; leu; tyr |
| Ser | thr |
| Thr | ser |
| Trp | tyr |
| Tyr | trp; phe |
| Val | ile; leu |

Substantial changes in function or immunological identity are made by selecting substitutions that are less conservative than those in Table 1, i.e., selecting residues that differ more significantly in their effect on maintaining (a) the structure of the polypeptide backbone in the area of the substitution, for example as a sheet of helical conformation, (b) the charge or hydrophobicity of the molecule at the target site or (c) the bulk of the side chain. The substitutions in general expected to produce the greatest changes in MKP properties will be those in which (a) a hydrophilic residue, e.g. seryl or threonyl, is substituted for (or by) a hydrophobic residue, e.g. leucyl, isoleucyl, phenylalanyl, valyl, or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having such a side chain, e.g. glycine.

MKP nucleic acid is defined as RNA or DNA which encodes MKP or which hybridizes to such DNA and remains stably bound to it under stringent conditions and is greater than about 10 bases in length. Stringent conditions are defined as low ionic strength and high temperature for washing, for example, 0.15 M NaCl/0.015 M Sodium citrate/0.1% NaDodSo$_4$ at 50°C, or alternatively the presence of denaturing agents such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate and 42°C for hybridization. DNA encoding MKP is obtained from fetal tissue cDNA libraries, ordinarily liver or dissected brain regions, from cDNAs or genomic DNA of AML leukemic cells, or by in vitro synthesis. Hybridizing nucleic acid generally is obtained by in vitro synthesis. The original discovery of a DNA sequence for MKP was fortuitous, but it will be understood that one skilled in the art would be able to readily obtain MKP-encoding nucleic acid having been supplied with the nucleotide sequence encoding MKP. This most conveniently is accomplished by probing human cDNA or genomic libraries by labelled oligonucleotide sequences selected from Fig. 1 in accord with known criteria, among which is that the sequence should be of sufficient length and sufficiently unambiguous that false positives are minimized. Typically, a P32 labelled oligonucleotide having about 30 to 50 bases is sufficient, particularly if the oligonucleotide contains one or more codons for methionine or tryptophane.

Of particular interest is MKP nucleic acid that encodes the full length molecule, including but not necessarily its native signal sequence. This is obtained by screening cDNA or genomic libraries using the full length clone depicted in Fig. 1 and, if necessary, using conventional primer extension procedures in order to secure DNA which is complete at its 5′ coding end. Such a clone is readily identified by the presence of a start codon in reading frame with the Fig. 1 sequence. The full length clone most likely also will encode a signal sequence of about 15 to 25 largely hydrophobic residues immediately following the start codon. The full length clone then is transfected into a mammalian host cell under the control of appropriate expression control sequences, whereupon the precursor will be processed and secreted as mature full length MKP. The N-terminus of this molecule is determined by conventional Edman degradation or the like to elucidate the mature amino terminus. This, together with an analysis of the C-terminal end of the native signal sequence, guides the selection of the proper N-terminal site for constructing heterologous signal sequence fusions usable, for example, in microbial host-vector systems.

The MKP-encoding nucleic acid is then ligated into a replicable vector for further cloning or for expression. Vectors are useful for performing two functions in collaboration with compatible host cells (a host-vector system). One function is to facilitate the cloning of the nucleic acid that encodes the MKP, i.e., to produce usable quantities of the nucleic acid. The other function is to direct the expression of MKP. One or both of these functions are performed by the vector-host system. The vectors will contain different components depending upon the function they are to perform as well as the host cell that is selected for cloning or expression.

Each vector will contain nucleic acid that encodes MKP as described above. Typically, this will be DNA that encodes the MKP in its mature form linked at its amino terminus to a secretion signal. This secretion signal preferably is the MKP presequence that normally directs the secretion of MKP from human cells in vivo. However, suitable secretion signals also include signals from other animal MKP, viral signals or signals from secreted polypeptides of the same or related species.

Expression and cloning vectors contain a nucleic acid sequence that enables the vector to replicate in one or more selected host cells. Generally, in cloning vectors this sequence is one that enables the vector to replicate independently of the host chromosomes, and includes origins of replication or autonomously replicating sequences. Such sequences are well-known for a variety of bacteria, yeast and viruses. The origin of replication from the well-known plasmid pBR322 is suitable for most gram negative bacteria, the 2µ plasmid origin for yeast and various viral origins (SV40, polyoma, adenovirus, VSV or BPV) are useful for cloning vectors in mammalian cells. Origins are not needed for mammalian expression vectors (the SV40 origin is used in the Examples only because it contains the early promoter). Most expression vectors are "shuttle" vectors, i.e. they are capable of replication in at least one class of organisms but can be transfected into another organism for expression. For example, a vector is cloned in E. coli and then the same vector is transfected into yeast or mammalian cells for expression even though it is not capable of replicating independently of the host cell chromosome.

DNA also is cloned by insertion into the host genome. This is readily accomplished with bacillus species, for example, by including in the vector a DNA sequence that is complementary to a sequence found in bacillus genomic DNA. Transfection of bacillus with this vector results in homologous recombination with the genome and insertion of MKP DNA. However, the recovery of genomic DNA encoding MKP is more complex than that of an exogenously replicated vector because restriction enzyme digestion is required to excise the MKP DNA.

Expression and cloning vectors should contain a selection gene, also termed a selectable marker. This is a gene that encodes a protein necessary for the survival or growth of a host cell transformed with the vector. The presence of this gene ensures that any host cell which deletes the vector will not obtain an advantage in growth or reproduction over transformed hosts. Typical selection genes encode proteins that (a) confer resistance to antibiotics or other toxins, e.g. ampicillin, neomycin, methotrexate or tetracycline, (b) complement auxotrophic deficiencies, or (c) supply critical nutrients not available from complex media, e.g. the gene encoding D-alanine racemase for bacilli.

A suitable selection gene for use in yeast is the trp1 gene present in the yeast plasmid YRp7 (Stinchcomb et al., 1979, "Nature" 282:39; Kingsman et al., 1979, "Gene" 7:141; or Tschemper et al., 1980, "Gene" 10:157). The trp1 gene provides a selection marker for a mutant strain of yeast lacking the ability to grow in tryptophan, for example ATCC No. 44076 or PEP4-1 (Jones, 1977, "Genetics" 85:12). The presence of the trp1 lesion in the yeast host cell genome then provides an effective environment for detecting transformation by growth in the absence of tryptophan. Similarly, Leu2 deficient yeast strains (ATCC 20,622 or 38,626) are complemented by known plasmids bearing the Leu2 gene.

Examples of suitable selectable markers for mammalian cells are dihydrofolate reductase (DHFR) or thymidine kinase. Such markers enable the identification of cells which were competent to take up the MKP nucleic acid. The mammalian Cell transformants are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes MKP. Amplification is the process by which genes in greater demand for the production of a protein critical for growth are reiterated in tandem within the chromosomes of successive generations of recombinant cells. Increased quantities of MKP are synthesized from the amplified DNA.

For example, cells transformed with the DHFR selection gene are first identified by culturing all of the transformants in a culture medium which lacks hypoxanthine, glycine, and thymidine. An appropriate host cell in this case is the Chinese hamster ovary (CHO) cell line deficient in DHFR activity, prepared and propagated as described by Urlaub and Chasin, 1980, "Proc. Nat'l. Acad. Sci. USA" 77:4216. A particularly useful DHFR is a mutant DHFR that is highly resistant to MTX (EP 117,060A). This selection agent can be used with any otherwise suitable host, e.g. ATCC No. CCL61 CHO-K1, notwithstanding the presence of endogenous DHFR. The DHFR and MKP-encoding DNA then is amplified by exposure to an agent (methotrexate, or MTX) that inactivates the DHFR. One ensures that the cell requires more DHFR (and consequently amplifies all exogenous DNA) by selecting only for cells that can grow in successive rounds of ever-greater MTX concentration.

Other methods, vectors and host cells suitable for adaptation to the synthesis of the hybrid receptor in recombinant vertebrate cell culture are described in M.J. Gething et al., "Nature" 293:620-625 (1981); N. Mantei et al., "Nature" 281:40-46 (1979); and A. Levinson et al., EP 117,060A and 117,058A. A particularly useful starting plasmid for mammalian cell culture expression of MKP is pE342.HBV E400.D22 (also called pE348HBVE400D22, EP 117,058A).

Expression vectors, unlike cloning vectors, should contain a promoter which is recognized by the host organism and is operably linked to the MKP nucleic acid. Promoters are untranslated sequences located upstream from the start codon of a structural gene (generally within about 100 to 1000 bp) that control the transcription and translation of nucleic acid under their control. They typically fall into two classes, inducible and constitutive. Inducible promoters are promoters that initiate increased levels of transcription from DNA under their control in response to some change in culture conditions, e.g. the presence or absence of a nutrient or a change in temperature. At this time a large number of promoters recognized by a variety of potential host cells are well known. These promoters are operably linked to MKP-encoding DNA by removing them from their gene of origin by restriction enzyme digestion, followed by insertion 5' to the start codon for MKP. This is not to say that the genomic MKP promoter is not usable. However, heterologous promoters generally will result in greater transcription and higher yields of expressed MKP.

Nucleic acid is operably linked when it is placed into a functional relationship with another nucleic acid sequence. For example, DNA for a presequence or secretory leader is operably linked to DNA for a polypeptide if it is expressed as a preprotein which participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, operably linked means that the DNA sequences being linked are contiguous and, in the case of a secretory leader, contiguous and in reading phase. Linking is accomplished by ligation at convenient restriction sites. If such sites do not exist then synthetic oligonucleotide adaptors or linkers are used in accord with conventional practice.

Promoters suitable for use with prokaryotic hosts include the $\beta$-lactamase and lactose promoter systems (Chang et al., 1978, "Nature" 275:615; and Goeddel et al., 1979, "Nature" 281:544), alkaline phosphatase, a tryptophan (trp) promoter system (Goeddel, 1980, "Nucleic Acids Res." 8:4057 and EPO Appln. Publ. No. 36,776) and hybrid promoters such as the tac promoter (H. de Boer et al., 1983, "Proc. Nat'l. Acad. Sci. USA" 80:21-25). However, other known bacterial promoters are suitable. Their nucleotide sequences have been published, thereby enabling a skilled worker operably to ligate them to DNA encoding MKP (Siebenlist et al., 1980, "Cell" 20:269) using linkers or adaptors to supply any required restriction sites. Promoters for use in bacterial systems also will contain a Shine-Dalgarno (S.D.) sequence operably linked to the DNA encoding MKP.

Suitable promoting sequences for use with yeast hosts include the promoters for 3-phosphoglycerate kinase (Hitzeman et al., 1980, "J. Biol. Chem." 255:2073) or other glycolytic enzymes (Hess et al., 1968, "J. Adv. Enzyme Reg." 7:149; and Holland, 1978, "Biochemistry" 17:4900), such as enolase, glyceraldehyde-3-phosphate dehydrogenase, hexokinase, pyruvate decarboxylase, phosphofructokinase, glucose-6-phosphate

EP 0 354 989 B1

isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., EP 73,657A. Yeast enhancers also are advantageously used with yeast promoters.

MKP transcription from vectors in mammalian host cells is controlled by promoters obtained from the genomes of viruses such as polyoma, cytomegalovirus, adenovirus, retroviruses, hepatitis-B virus and most preferably Simian Virus 40 (SV40), or from heterologous mammalian promoters, e.g. the actin promoter. The early and late promoters of the SV40 virus are conveniently obtained as an SV40 restriction fragment which also contains the SV40 viral origin of replication (Fiers et al., 1978, "Nature" 273:113). Of course, promoters from the host cell or related species also are useful herein.

Transcription of MKP-encoding DNA by higher eukaryotes is increased by inserting an enhancer sequence into the vector. An enhancer is a nucleotide sequence, usually about from 10-300 bp, that acts on a promoter to increase its transcription and does so in a manner that is relatively orientation and position independent. Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenoviral enhancers. The enhancer may be spliced into the vector at a position 5′ or 3′ to the MKP-encoding sequence, but is preferably located at a site 5′ from the promoter.

Expression vectors used in eukaryotic host cells (yeast, fungi, insect, plant, animal, human or nucleated cells from other multicellular organisms) will also contain sequences necessary for the termination of transcription and for stabilizing the mRNA. Such sequences are commonly available from the 5′ and, occasionally 3′ untranslated regions of eukaryotic or viral DNAs or cDNAs. These regions contain regions that are transcribed as polyadenylated segments in the untranslated portion of the mRNA encoding MKP. The 3′ untranslated regions also include transcription termination sites.

Suitable host cells for cloning or expressing the vectors herein are the prokaryote, yeast or higher eukaryote cells described above. Suitable prokaryotes include gram negative or gram positive organisms, for example E. coli or bacilli. A preferred cloning host is E. coli 294 (ATCC 31,446) although other gram negative or gram positive prokaryotes such as E. coli B, E. coli X1776 (ATCC 31,537), E. coli W3110 (ATCC 27,325), pseudomonas species, or Serratia Marcesans are suitable.

In addition to prokaryotes, eukaryotic microbes such as filamentous fungi or yeast are suitable hosts for MKP-encoding vectors. Saccharomyces cerevisiae, or common baker's yeast, is the most commonly used among lower eukaryotic host microorganisms. However, a number of other genera, species and strains are commonly available and useful herein.

Suitable host cells for the expression of MKP are derived from multicellular organisms. Such host cells are capable of complex processing and glycosylation activities. In principle, any higher eukaryotic cell culture is workable, whether from vertebrate or invertebrate culture, although cells from mammals such as humans are preferred. Propagation of such cells in culture is per se well known. See Tissue Culture, Academic Press, Kruse and Patterson, editors (1973). Examples of useful mammalian host cell lines are VERO and HeLa cells, Chinese hamster ovary cell lines, the WI38, BHK, COS-7, MDCK cell lines and human embryonic kidney cell line 293.

Host cells are transformed with the above-described expression or cloning vectors and cultured in conventional nutrient media modified as is appropriate for inducing promoters or selecting transformants containing amplified genes. The culture conditions, such as temperature, pH and the like, suitably are those previously used with the host cell selected for cloning or expression, as the case may be, and will be apparent to the ordinary artisan.

MKP preferably is recovered from the culture medium as a secreted protein, although it also may be recovered from host cell lysates when directly expressed without a secretory signal. As a first step, the culture medium or lysate is centrifuged to remove particulate cell debris. MKP also is purified from contaminant soluble proteins for example by fractionation on immunoaffinity or ion exchange columns. Such processes as chromatography on alkyl Sepharose, silica or an anion or cation exchange resin or gel electrophoresis are used to separate the MKP from contaminants. MKP variants in which residues have been deleted, inserted or substituted are recovered in the same fashion as native MKP, taking account of any substantial changes in properties occasioned by the variation. For example, preparation of an MKP fusion with another protein, e.g. bacterial antigens, facilitates purification because an immunoaffinity column containing antibody to the antigen can be used to adsorb the fusion. A protease inhibitor such as PMSF also may be useful to inhibit proteolytic degradation during purification, and antibiotics may be included to prevent the growth of adventitious contaminants. One skilled in the art will appreciate that purification methods suitable for native MKP may require modification to account for changes in the character of MKP or its variants upon expression in recombinant cell culture.

Therapeutic formulations of MKP are prepared for storage by mixing MKP having the desired degree of purity with optional physiologically acceptable carriers, excipients or stabilizers in the form of lyophilized cake or aqueous solutions. Acceptable carriers, excipients or stabilizers are nontoxic to recipients at the dosages and concentrations employed, and include buffers such as phosphate citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone amino acids such as glycine, glutamic acid or aspartic acid; monosaccharides, disaccharides and other carbohydrates including glucose, mannose, or dextrins; chelating agents such as EDTA; sugar alcohols such as mannitol or sorbitol; salt-forming counterions such as sodium, and/or nonionic surfactants such as Tween, Pluronics or PEG. MKP to be used for therapeutic administration must be sterile. This is readily accomplished by filtration through sterile filtration membranes, prior to or following lyophilization. MKP ordinarily will be stored in lyophilized form. The pH of the MKP preparations typically will be about from 6 to 8.

Therapeutic MKP compositions generally are placed into a container having a sterile access port, for example, an intravenous solution bag or vial having a stopper pierceable by a hypodermic injection needle.

MKP optionally is combined with or administered in concert with other thrombo- or erythro-potentiating agents including interleukin-3, erythropoietin or activin, and is used with other conventional therapies for thrombocytopenia.

Antibodies to MKP are used with other conventional therapies for thrombocytemia, where they block the abnormally high megakaryocytopoiesis rates characterizing this type of syndrome.

MKP preparations typically are administered to animals exhibiting thrombocytopenia.. The route of administration is in accord with known methods, e.g. intravenous, intraperitoneal, intramuscular, intralesional infusion or injection, or by sustained release systems as noted below. MKP is administered continuously by infusion, although bolus injection is acceptable.

Suitable examples of sustained release preparations include semipermeable polymer matrices in the form of shaped articles, e. g. films, or microcapsules. Sustained release matrices include polylactides (U.S. Patent 3,773,919, EP 58,481), copolymers of L-glutamic acid and gamma ethyl-L-glutamate (U. Sidman et al., 1983, "Biopolymers" 22 (1): 547-556), poly (2-hydroxyethyl-methacrylate) (R. Langer, et al., 1981, "J. Biomed. Mater. Res." 15: 167-277 and R. Langer, 1982, Chem. Tech." 12: 98-105), ethylene vinyl acetate (R. Langer et al., Id.) or poly-D-(-)-3-Hydroxybutyric acid (EP 133,988A). Sustained release MKP compositions also include liposomally entrapped MKP. Liposomes containing MKP are prepared by methods known per se: DE 3,218,121A; Epstein et al., 1985, "Proc. Natl. Acad. Sci. USA" 82: 3688-3692; Hwang et al., 1980, "Proc. Natl. Acad. Sci. USA" 77: 4030-4034; EP 52322A; EP 36676A; EP 88046A; EP 143949A; EP 142641A; Japanese patent application 83-118008; U.S. patents 4,485,045 and 4,544,545; and EP 102,324A. Ordinarily the liposomes are of the small (about 200-800 Angstroms) unilamelar type in which the lipid content is greater than about 30 mol. % cholesterol, the selected proportion being adjusted for the optimal MKP therapy.

The amount of MKP to be employed therapeutically will depend, for example, upon the therapeutic objectives, the route of administration, and the condition of the patient. Accordingly, it will be necessary for the therapist to titer the dosage and modify the route of administration as required to obtain the optimal therapeutic effect. Typically, the clinician will administer MKP until a dosage is reached that reestablishes a normal platelet level of about $250 \pm 50 \times 10^3/\mu l$ of blood. The progress of this therapy is easily monitored by conventional hematology assays or laboratory cell counts. Suitable starting therapeutic doses can be extrapolated from the in vitro efficacy studies described above.

Suitable diagnostic assays for MKP and its antibodies are well known per se. In addition to the bioassay described below, one may employ competitive, sandwich and steric inhibition immunoassay techniques. The competitive and sandwich methods employ a phase separation step as an integral part of the method while steric inhibition assays are conducted in a single reaction mixture. Fundamentally, the same procedures are used for the assay of MKP and for substances that bind MKP, although certain methods will be favored depending upon the molecular weight of the substance being assayed. Therefore, the substance to be tested is referred to herein as an analyte, irrespective of its status otherwise as an antigen or antibody, and proteins which bind to the analyte are denominated binding partners, whether they be antibodies, cell surface receptors or antigens.

Analytical methods for MKP or its antibodies all use one or more of the following reagents: labelled analyte analogue, immobilized analyte analogue, labelled binding partner, immobilized binding partner and steric conjugates. The labelled reagents also are known as "tracers".

The label used is any detectable functionality which does not interfere with the binding of analyte and its binding partner. Numerous labels are known for use in immunoassay, examples including enzymes such as horseradish peroxidase, radioisotopes such as $^{14}C$ and $^{131}I$, fluorophores such as rare earth chelates or fluorescein, stable free radicals and the like. Conventional methods are available to covalently bind these labels to proteins or polypeptides. Such bonding methods are suitable for use with MKP or its antibodies, all of which are proteinaceous.

Immobilization of reagents is required for certain assay methods. Immobilization entails separating the binding partner from any analyte which remains free in solution. This conventionally is accomplished by either insolubilizing the binding partner or analyte analogue before the assay procedure, as by adsorption to a water insoluble matrix or surface (Bennich et al., U.S. 3,720,760), by covalent coupling (for example using glutaraldehyde cross-linking), or by insolubilizing the partner or analogue afterward, e.g., by immunoprecipitation.

Other assay methods, known as competitive or sandwich assays, are well established and widely used in the commercial diagnostics industry.

Competitive assays rely on the ability of a labelled analogue (the "tracer") to compete with the test sample analyte for a limited number of binding sites on a common binding partner. The binding partner generally is insolubilized before or after the competition and then the tracer and analyte bound to the binding partner are separated from the unbound tracer and analyte. This separation is accomplished by decanting (where the binding partner was preinsolubilized) or by centrifuging (where the binding partner was precipitated after the competitive reaction). The amount of test sample analyte is inversely proportional to the amount of bound tracer as measured by the amount of marker substance. Dose-response curves with known amounts of analyte are prepared and compared with the test results in order to quantitatively determine the amount of analyte present in the test sample. These assays are called ELISA systems when enzymes are used as the detectable markers.

Another species of competitive assay, called a "homogeneous" assay, does not require a phase separation. Here, a conjugate of an enzyme with the analyte is prepared and used such that when anti-analyte binds to the analyte the presence of the anti-analyte modifies the enzyme activity. In this case, MKP or its immunologically active fragments are conjugated with a bifunctional organic bridge to an enzyme such as peroxidase. Conjugates are selected for use with anti-MKP so that binding of the anti-MKP inhibits or potentiates the enzyme activity of the label. This method per se is widely practiced under the name of EMIT.

Steric conjugates are used in steric hindrance methods for homogeneous assay. These conjugates are synthesized by covalently linking a low molecular weight hapten to a small analyte so that antibody to hapten substantially is unable to bind the conjugate at the same time as anti-analyte. Under this assay procedure the analyte present in the test sample will bind anti-analyte, thereby allowing anti-hapten to bind the conjugate resulting in a change in the character of the conjugate hapten, e.g., a change in fluorescence when the hapten is a fluorophore. Due to the large molecular weight of MKP, homogeneous or steric hinderance methods are not preferred for the determination of MKP.

Sandwich assays particularly are useful for the determination of MKP or MKP antibodies. In sequential sandwich assays an immobilized binding partner is used to adsorb test sample analyte, the test sample is removed as by washing, the bound analyte is used to adsorb labelled binding partner and bound material then separated from residual tracer. The amount of bound tracer is directly proportional to test sample analyte. In "simultaneous" sandwich assays the test sample is not separated before adding the labelled binding partner. A sequential sandwich assay using an anti-MKP monoclonal antibody as one antibody and a polyclonal anti-MKP antibody as the other is useful in testing samples for MKP activity.

The foregoing are merely exemplary diagnostic assays for MKP and antibodies. Other methods now or hereafter developed for the determination of these analytes are included within the scope hereof, including the bioassay described infra.

Covalent modifications of the MKP molecule are included within the scope hereof. Such modifications are introduced into the molecule by reacting targeted amino acid residues of the purified or crude protein with an organic derivatizing agent that is capable of reacting with selected side chains or terminal residues. The resulting covalent derivatives are useful as immunogens or in programs directed at identifying residues important for biological activity.

Cysteinyl residues most commonly are reacted with $\alpha$-haloacetates (and corresponding amines), such as chloracetic acid or chloroacetamide to give, carboxymethyl or carboxamidomethyl derivatives. Cysteinyl residues also are derivatized by reaction with bromotrifluoroacetone, $\alpha$-bromo-$\beta$-(5-imidozoyl) propionic acid chloroacetol phosphate, N-alkylmaleimides, 3-nitro-2-pyridyl disulfide, methyl 2-pyridyl disulfide, p-chloromercuribenzoate 2-chloromercuri-4-nitrophenol or chloro-7-nitrobenzo-2-oxa-1, 3-diazole.

Histidyl residues are derivatized by reaction with diethylpyrocarbonate at pH 5.5 to 7.0; this agent is relatively specific for the histidyl side chain. Parabromophenacyl bromide also is useful; the reaction should be performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing $\alpha\nu$ amino-containing residues include imidoesters such as methyl picolinimidate; pyridoxal phosphate; pyridoxal; claroborohydride; trinitrobenzenesulfonic acid; O-methylisourea; 2,4-pentanedione; and transaminase-catalyzed reaction with glyoxylate.

Arginyl residues are modified by reaction with one of several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine $\epsilon$-amino group.

The specific modification of tyrosyl residues per se has been extensively studied, with particular interest in introducing spectral labels into tyrosyl residues by reaction vith aromatic diazonium compounds or tetranitromethane. Most commonly, N-acetylimidizol and tetranitromethane are used to form O-acetyl tyrosyl species and 3-nitro derivatives, respectively. Tyrosyl residues are iodinated using $^{125}$I or $^{131}$I to prepare labelled proteins for use in radioimmunoassay, the chloramine T method described above being suitable.

Carboxyl side groups (aspartyl or glutamyl) are selectively modified by reaction uith carbodiimides (R′-N-C-N-R′) such as 1-cyclohexyl-3-(2-morpholinyl-(4)-ethyl) carbodiimide or 1-ethyl-3-(4-azonia-4,4-dimethylpentyl)-carbodiimide. Furthermore, aspartyl and glutamyl residues are converted to asparaginyl and glutaminyl residues by reaction with ammonium ions.

Derivatization with bifunctional agents is useful for preparing intermolecular aggregates of the protein vith immunogenic polypeptides as well as for cross-linking the protein to a water insoluble support matrix or surface for use in the assay or affinity purification of MKP antibody. Commonly used cross-linking agents include 1,1-bis(diazoacetyl)-2-phenylethane, glutaraldehyde, N-hydroxysuccinimide esters, for example esters with 4-azidosalicylic acid, homobifunctional imidoesters including disuccinimidyl esters such as 3,3′-dithiobis (succinimidylpropionate) and bifunctional maleimides such as bis-N-maleimido-1,8-octane. Derivatizing agents such as methyl-3-[(p-azidophenyl)dithio] propioimidate yield photactivatable intermediates which are capable of forming cross-links in the presence of light. Alternatively, reactive water insoluble matrices such as cyanogren bromide activated carbohydrates and the reactive substrates described in U.S. patents 3,969,287; 3,691,016; 4,195,128; 4,247,642; 4,229,537 and 4,330,440 are employed for protein immobilization.

Glutaminyl and asparaginyl residues are frequently deamidated to the corresponding glutamyl and aspartyl residues. Alternatively, these residues are deamidated under mildly acidic conditions. Either form of these residues falls within the scope of MKP.

Other modifications include hydroxylation of proline and lysine, phosphorylation of hydroxyl groups of seryl or threonyl residues, methylation of the $\alpha$-amino groups of lysine, arginine, and hystidine side chains (T.E. Creighton, Proteins: Structure and Molecular Properties, W.H. Freeman & Co., San Francisco pp 79-86 [1983]), acetylation of the N-terminal amine and, in some instances, amidation of the C-terminal carboxyl.

Antibodies to MKP generally are raised in animals by multiple sc or ip injections of MKP and an adjuvant such as Freund's adjuvant and/or tumor necrosis factor. It may be useful to conjugate the MKP to a protein which is immunogenic in the species to be immunized, e.g., keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glutaraldehyde, succinic anhydride, $SOCl_2$ or $R^1N - C - NR$. Also, aggregating agents such as alum may be used to enhance the immune response.

Animals, ordinarily rabbits or mice, are immunized against the immunogenic conjugates or derivatives, preferably a glutaraldehyde or succinic anhydride conjugate to soybean trypsin inhibitor, by combining 1 mg or 1 $\mu$g of conjugate (for rabbits or mice, respectively) with 3 volumes of Freund's complete adjuvant and injecting the solution intradermally at multiple sites with human TNF-$\alpha$ at ____ $\mu$g/Kg. One month later the animals are boosted with 1/5 to 1/10 the original amount of conjugate in Freund's complete adjuvant by subcutaneous injection at multiple sites. 7 to 14 days later animals are bled and the serum assayed for anti-MKP titer. Animals are boosted until the titer plateaus. Preferably, the animal is boosted with the conjugate of the same MKP polypeptide, but conjugated to a different protein and/or through a different cross-linking agent.

Monoclonal antibodies are prepared by recovering spleen cells from appropriate immunized animals and immortalizing the cells in conventional fashion, e.g. by fusion with myeloma cells or by EB virus transformation and screening for clones expressing the desired antibody.

Quantification of amplified MKP genes in peripheral blood cells of leukemia patients is diagnosed by DNA dot blot hybridization. For this purpose, serial dilutions of denatured genomic DNa purified by standard procedures from blood samples of tested patients are spotted onto nylon filters using appropriate applicators. Purified MKP DNA is being spotted in parallel for calibration. All samples also contain denatured carrier DNA such as salmon sperm DNA to yield a total constant amount of DNA per spot (usually, 2 $\mu$g). Filters are incubated for hybridization with P32 MKP DNA or with biotinylated MKP DNA and washed under stringent conditions. Quantities of genomic MKP DNA that hybridize with the labeled probe in each patient are then determined in values equivalent to pg of MKP DNA by optical densitometry of exposed X-ray film (in the case of P32-labeled probe) or of conjugated second fluoroprobe (in the case of biotinylated MKP DNA). In a typical diagnostic test, samples of up to 2 $\mu$g of genomic DNA are hybridized for 16 hr. and washed as detailed above.

The principle of this assay is the detection of putative perturbations at the level of the MKP gene and not the protein product. Since molecular changes at the level of genes and chromosomes appear to be linked to the occurrence if congenital syndromes as well as to reflect the severeness of certain diseases, this type of measurement can add novel clinical information.

Example 1

A modification of Iscove's method (in Hematopoietic Cell Differentiation, ICN-UCLA Symposia on Molecular and Cellular Biology, Vol. 10, Golde et al., eds. [New York, Academy Press], pp 37-52) for growing hemopoietic stem cells in methyl cellulose culture was used combined with IMDM (Iscove's modification of Dulbecco's medium) medium. Bone marrow (BM) cells from 8-12 week-old C3H/HeJ (endotoxin-resistant) mice were ejected by syringe and grown in the above medium at a concentration of $10^5$ cells/ml at 5% $CO_2$ and 37°C.

5% PWM-Sp-CM + 10% WEHI-CM constituted the positive control while 10% WEHI-CM alone was the negative control for all subsequent experiments. In a typical experiment the number of megakaryocytic colonies scored at seven days in the positive control was 60/ml/plate while 30/ml/plate were noted in the negative control.

I.

A novel cDNA sequence was identified in reverse transcripts of fetal ganglioside mRNA by the use of BuChE probes. This consisted of the exact first 249 nucleotides of BuChE cDNA (Prody et al., 1987) followed by the sequence included in figure 1. The connecting site therefore was

```
... TTT CTT GGA ATT CGC GGC ...
    Phe Leu Gly Ile Arg Gly

    BuChE cDNA   Clone 14 cDNA
```

Note, however, that it is possible that the ATT triplet was contributed also from BuChE cDNA and is not part of clone 14.

Xenopus oocytes were injected with either clone 14 nonsense mRNA, clone 14 sense mRNA, Butyrylcholinesterase (BuChE) mRNA or Barth medium (the medium used for growing the oocytes). The supernatants were harvested after 20-22 hours, diluted with IMDM to 1:20, filter-sterilized and diluted once more to 1:100 with IMDM. Each of the supernatants was introduced at two concentrations, 1:20 and 1:100, into culture media containing only WEHI-CM. In each case they constituted 5% of the total volume. Megakaryocytic colonies were scored after seven days of incubation.

| Factors Added | No. of Megakaryocytic Colonies/ml[a] | | | |
| --- | --- | --- | --- | --- |
| | Positive | Negative Results | Inhibitory | N[b] |
| 1. PWM + WEHI | 63±4 | | | 17 |
| 2. WEHI | | 28±3 | | 8 |
| 3. 14 nonsense, 1:20 | | 54±4 | | 4 |
|     ", 1:100 | | 36±8 | | 2 |
| 4. 14 sense, 1:20 | 68±1 | | | 3 |
|     ", 1:100 | 64±9 | | | 4 |
| 5. BuChE, 1:20 | | | 11±5 | 2 |
|     " 1:100 | | | 16±4 | 2 |
| 6. Barth, 1:20 | 91±4 | | | 4 |
|     " 1:100 | 67±8 | | | 4 |

a. Results expressed in mean ±SEM.

b. N - No. of plates scored.

From this experiment it was evident that both the 14 sense clone and the Barth medium produced results similar to or superior to the synergistic effect of PWM-SpCM and WEHI-CM while BuChE was distinctly inhibitory of megakaryocytic colony growth. This latter finding is in keeping with the earlier results of Burstein et al. (J. Cell Physiol. 103:201-208 [1980]) which demonstrated that inhibitor to acetylcholinesterase (AChE) stimulated megakaryocytic colony growth. The 14 nonsense clone gave intermediate values which were difficult to interpret. At this point, we could not distinguish between the background effect of the endogenous oocyte secretions in the Barth medium and those induced by the 14 sense clone when injected into the oocytes. It should be noted in this respect that mRNA microinjection reduces the endogenous level of protein secretion from oocytes (H. Soreq, unpublished). It is hence possible that the clone 14 nonsense group is the correct negative control for the 14 sense clone.

II.

In order to determine whether the surrounding cells of the oocytes were producing factors active in potentiating megakaryocytic development the above experiment was repeated using whole oocytes as before and in addition oocytes depleted of surrounding cells by collagenase treatment (collagenase Type I, Sigma). Both types of oocytes were injected with 14 sense clone. The results corroborated those of Experiment I which were repeated here, including the 14 sense and antisense clones, BuChE, and the Barth-injected oocyte CM.

| Factors added | Positive Results | N |
| --- | --- | --- |
| 14 sense, collagenase-treated, 1:20 | 96±18 | 2 |
| 14 sense, collagenase-treated, 1:100 | 38±2 | 2 |

The number of megakaryocytic colonies produced from the CM of the collagenase-treated oocytes injected with 14 sense clone were equivalent to those produced from the Barth medium and slightly higher than those formed from the untreated 14 sense clone-oocytes and the PWM + WEHI controls. It was therefore tentatively concluded that the surrounding cells of the oocytes were not contributing any additional growth factors to those produced by the Barth-injected oocytes and/or the 14 sense clone mRNA.

III.

In another attempt to distinguish between the possible effects of native secretions of the oocyte and those of the 14 sense clone, the diluted supernatants of untreated oocytes and Barth-injected oocytes were treated for 3′ and 15′ at 45°C. The rationale here being that amphibian proteins are far more sensitive to temperatures such as 45°C than are mammalian proteins and would possibly be inactivated. Care was taken to dilute the supernatants before heating so that concentration effects would not mask those due to heating. The results are as tabulated:

| Factors Added | No. of Megakaryocytic Colonies/ml | N |
|---|---|---|
| 1. PWM + WEHI<br>2. WEHI | 63±3<br>28±3 | 17<br>8 |
| 3. Untreated Oocyte Secretion | | |
| 1:20, 3′ incubation<br>1:20, 15″ incubation<br>1:100, 15′ incubation | 32±4<br>20±4<br>26±7 | 2<br>4<br>4 |
| 4. Barth-treated Oocyte Secretion | | |
| 1:100, 3′ incubation<br>1:20, 15′ incubation<br>1:100, 15′ incubation | 36±4<br>27±5<br>42±8 | 2<br>4<br>4 |

It was concluded from the above experiment that heating oocyte supernatants for even 3′ causes a significant reduction in megakaryocyte-potentiating activity. Both Barth-injected and untreated oocyte secretions contain this activity in similar amounts, demonstrating that the native oocyte factor is not a result of the injection itself.

IV.

The effect of CM's from oocytes injected with 14 sense clone or Barth medium before and after collagenase treatment on the stimulation of early multipotential hemopoietic precursors was tested. Cultures containing erythropoietin (EPO) and WEHI-3-CM were used as the basis of comparison for all experimental combinations. This combination of factors is synergistic and is known to produce a fair number of large mixed colonies, i.e., clonal colonies containing several types of hemopoietic cells. In this case, the predominant cell type is erythroid, with smaller numbers of macrophages, granulocytes and megakaryocytes.

Two units of EPO/ml were used in these experiments and the incubation period was for 8-10 days at 5% $CO_2$ and 37°C.

| Factors Added | Mixed Colonies/ml | G-M Colonies/ml | N |
|---|---|---|---|
| EPO + WEHI<br>EPO + 14 sense, 1:20<br>WEHI + 14 sense, 1:20 | 19±1<br>0<br>0 | 22<br>0<br>111 | 2<br>2<br>2 |
| EPO + WEHI + 14 sense | | | |
| 1:20<br>1:100<br>EPO + WEHI + 14 sense collagenase-treated 1:20<br>EPO + WEHI + Barth 1:100 | 30±2<br>17±3<br>34±3<br>37±0.5 | n.c.<br>n.c.<br>n.c.<br>n.c. | 2<br>2<br>2<br>2 |

It was concluded from this experiment that CM's from Barth and 14 sense clone contain a hemopoietic growth factor, that in the presence of both EPO and WEHI-CM enhances synergistically the production of mixed colonies containing predominantly cells of the erythroid lineage. The effect was most pronounced at the 1:20 dilution. Here, as in the megakaryocytic experiments, there was no difference between oocytes with or without surrounding cells. Once again, it was not possible to distinguish between the effect of the native oocyte secretion and that induced by the injection of the 14 sense clone. From the few observations made, it would seem that the 14 sense clone also had a pronounced effect on the number of granulocyte-macrophage colonies produced.

V.

Morphological Studies

Cytospin preparations were made of colonies appearing in cultures produced under varying conditions, using a Shandon, Model 2 cytocentrifuge and May-Grunwald-Giemsa stain. Colonies were found to contain a large proportion (up to 80%) of megakaryocytes, both early and mature, and a smaller proportion of granulocytes, macrophages and plasmacytes. Early megakaryocytes, characterized by single, large, lobed or kidney-shaped nuclei, were much more abundant than the mature forms.

Using differential interference contrast, an interesting group of phenomena was observed. At given stages of development of the cultures (at 6-10 days), tremendous amoeboid-like forms were seen to move-flow through the medium, frequently covering the whole field of vision. These free-form megakaryocytes, probably the result of fusion, would within minutes begin to pinch off into two smaller megakaryocytes. At times, one could even observe a simultaneous pinching-off at two separate points, resulting in the formation of three smaller megakaryocytes. Miniature density gradients were formed in the cultures where numerous cell-like forms in the same size range as platelets (0.5 - 1 micron) were found at the bottom of the Petri dishes. At intermediate levels, small and medium-sized megakaryocytes would be concentrated and at the top floated the tremendous free-form megakaryocytes. Frequently, clusters of granulocytes would be attached to the surface of the intermediate-sized megakaryocytes. It is possible that the pinching-off of smaller megakaryocytes from the larger free-forms may be part of the non-mitotic, cytoplasmic maturation of megakaryopoiesis.

Conclusions

1. The synergistic combination of PWM-SpCM + WEHI-3-CM and WEHI-3-CM alone can be used as megakaryocyte potentiators in a methyl cellulose-IMDM clonal assay and serve as a basis of comparison of megakaryocytopoietic activity of unknown growth factors. The sources of these growth factors are the supernatants of Xenopus oocytes induced by mRNAs of different clones developed from a cDNA library of human newborn brain stem.

2. Substitution of the PWM-SpCM by the various supernatants of the sense clone 14 and the Barth medium used for oocyte growth resulted in a similar synergistic potentiation of CFU-megakaryocyte growth. This left in doubt, however, the source of the synergistic factors.

3. BuChE mRNA, when translated into protein by Xenopus oocytes, inhibited the growth of CFU-megakaryocytes, behaving in this fashion behaving in this fashion inverseley to inhibitors of AChE, a result expected in view of inhibitor studies and the considerable sequence homology between human AChE and BuChE.

4. Oocyte surrounding cells were not responsible for the synergistic activity noted, as their removal by collagenase had, if any, an increased effect on megakaryocytic colony formation.

5. Heating of the supernatants of untreated or Barth-injected oocytes for 3-15″ at 45°C caused a significant reduction in megakaryocytic synergistic activity, indicating that at least some of the synergistic activity may have been due to temperature-sensitive amphibian proteins.

6. The same supernatants of the 14 clone and the Barth-injected oocytes proved to have a substantial synergistic action with EPO and WEHI-CM on mixed colony formation and CFU-GM formation. This activity, if found to be associated with the same molecule as that producing the synergistic action of CFU-megakaryocytic colonies, may be suggestive of the multi-CSF nature of the activity. In this context, EPO may have an effect on the development of the CFU-megakaryocytic colonies.

7. On the basis of previous investigations with WEHI-3-CM, it can be assumed that the action of WEHI in the above experiments is caused by the presence of IL-3 in this CM.

Example 2

The study in this was undertaken to investigate the assumption that clone 14 and the human cholinesterase genes are physically related and do not merely represent an artifactual recombination event, and that altered clone 14 genes (rearranged, amplified or mutagenized in any other way) are involved with the high platelet counts in acute myeloid leukemia patients.

17 cases of leukemia were therefore looked at, using butyryl cholinesterase (BuChE) cDNA and clone 14 cDNA as probes. These included 9 cases of AML, 5 cases of $AMLM_2$ (characterized by bad prognosis and rapid progress), one case of AMOL (acute monocytic leukemia), one case of AMGL (acute

EP 0 354 989 B1

megakaryocytic leukemia) and one with the potential diagnosis of polycytemia vera, a semi-malignant syndrome implicated with abnormal erythropoiesis. Minor modifications in the DNA restriction patterns could be observed in several of these, but were difficult to interpret. However, two of the $AMLM_2$ blood DNA samples displayed considerable amplification as follows:

| Sample No. | Amplified DNA Fragments (+Enzymes) | | |
|---|---|---|---|
| | $1070(AMLM_2)$ | Adr.$(=L_2)(AMLM_2)$ | Healthy Control |
| Labeled cDNA Probe | | | |
| Clone 14 | 3.8 Kb + + ($PVU_2$) 2.4 Kb + + ($PVU_2$) 1.8 Kb + + ($PVU_2$) 3.0 Kb + + + (HindIII) 2.0 Kb + + + (HindIII) 1.8 Kb + + + (HindIII) | 2.0 Kb + + (EcoRI) | + + + + |
| BuChEcDNA | 4.4 Kb + + + ($PVU_2$) + + 3.0 Kb + + + (HindIII) | 3.0 Kb + + + ($PVU_2$) 2.5 Kb + + + ($PVU_2$) 3.0 Kb + + + (EcoRI) 4.2 Kb + (EcoRI) 3.9 Kb + (EcoRI) | - 3.0 Kb + ($PVU_2$) 2.5 Kb + ($PVU_2$) - - - |

In Summary: amplified signals in both existing and non-detected restriction fragments were observed with 3 different enzymes ($PVU_2$, HindIII and EcoRI) in two different $AMLM_2$ cases out of 5. Most interestingly, the amplification of clone 14-hybridizable DNA was accompanied by amplification in BuChE positive sequences. The fact that different sizes of the restriction fragments hybridized with the 3′ probe excluded the possibility of cross-hybridization. Several of the amplified bands could be observed, with lower intensities, in the presumptive polycytemia vera (Vaguez disease) case, or in one or two of the other AML cases.

The adr. sample represents a patient, currently under remission following chemotherapy treatment, whose serum and erythrocyte ChE were apparently normal in levels and biochemical characteristics. ChE genes and clone 14 are both co-regulated and co-amplified under specific stimulants.

**Claims**

1. An isolate of a nucleic acid comprising nucleotides 1-564 of Fig. 1.

2. A nucleic acid having greater than 10 bp, which is capable of hybridizing under stringent conditions with the nucleic acid of claim 1 and encoding a protein having the ability to modulate the growth or differentiation of blood cells or their progenitor cells or the ability to cross-react with an antibody raised against said modulating protein, which stringent conditions are defined as washing in the presence of 0.15 M NaCl / 0.015 M sodium citrate / 0.1% $NaDoSO_4$ at 50°C or alternatively the presence of 50% (vol/vol) formamide with 0.1% bovine serum albumin / 0.1% Ficoll / 0.1% polyvinylpyrrolidone / 50 mM sodium phosphate buffer at pH 6.5 with 750 mM NaCl, 75 mM sodium citrate and 42°C for hybridization.

3. A replicable vector comprising a nucleic acid of claim 1 or 2.

4. A host cell transformed with a nucleic acid of claims 1 or 2.

5. The host cell of claim 4, which is a mammalian cell.

6. A method for diagnosis of leukemia comprising assaying a leukemia cell for transcription or translation of the nucleic acid of claim 1 or 2 or for detection of structural changes such as deletions, rearrangements or amplification of such nucleic acid.

7. Protein comprising an amino acid sequence encoded by the nucleic acid sequence of claim 1 or 2.

17

8. A composition comprising a cell-free protein of claim 7.

9. An antibody capable of binding the protein of claim 7.

10. A protein of claim 7 labeled with a detectable moiety.

11. The protein of claim 10 wherein the moiety is a radioisotope or enzyme.

12. A composition suitable for administration to a patient comprising a therapeutically effective amount of a protein of claim 7 and a physiologically acceptable carrier.

13. A composition suitable for administration to a patient comprising a therapeutically effective amount of antibodies to a protein of claim 7, capable of blocking its physiological activity, and an acceptable carrier.

**Patentansprüche**

1. Isolat einer Nukleinsäure, umfassend die Nukleotide 1 bis 564 von Figur 1.

2. Nukleinsäure von mehr als 10 bp, welche fähig ist unter stringenten Bedingungen mit der Nukleinsäure von Anspruch 1 zu hybridisieren und für ein Protein zu codieren mit der Fähigkeit das Wachstum oder die Differenzierung von Blutzellen oder deren Vorläuferzellen zu modulieren oder der Fähigkeit mit einem gegen das modulierende Protein erzeugten Antikörper kreuzzureagieren, wobei stringente Bedingungen definiert sind als Waschen in der Gegenwart von 0,15 M NaCl / 0,015 M Natriumcitrat / 0,1 % NaDoSO$_4$ bei 50°C oder alternativ die Gegenwart von 50 % (vol/vol) Formamid mit 0,1 % Rinderserumalbumin / 0,1 % Ficoll / 0,1 % Polyvinylpyrrolidon / 50 mM Natriumphosphatpuffer bei pH 6,5 mit 750 mM NaCl, 75 mM Natriumcitrat und 42°C für die Hybridisierung.

3. Replizierbarer Vektor, umfassend eine Nukleinsäure nach Anspruch 1 oder 2.

4. Wirtszelle, die mit einer Nukleinsäure nach Anspruch 1 oder 2 transformiert ist.

5. Wirtszelle nach Anspruch 4, die eine Säugerzelle ist.

6. Verfahren zur Diagnose von Leukämie, umfassend das Testen einer Leukämiezelle auf Transkription oder Translation der Nukleinsäure nach Anspruch 1 oder 2 oder auf Bestimmung struktureller Veränderungen wie etwa Deletionen, Umlagerungen oder Amplifizierung einer derartigen Nukleinsäure.

7. Protein, umfassend eine durch die Nukleinsäuresequenz nach Anspruch 1 oder 2 codierte Aminosäuresequenz.

8. Zusammensetzung, umfassend ein zellfreies Protein nach Anspruch 7.

9. Antikörper, der fähig ist das Protein nach Anspruch 7 zu binden.

10. Protein nach Anspruch 7, das mit einer nachweisbaren Gruppe markiert ist.

11. Protein nach Anspruch 10, wobei die Gruppe ein Radioisotop oder ein Enzym ist.

12. Zusammensetzung, geeignet zur Verabreichung an einen Patienten, umfassend eine therapeutisch wirksame Menge eines Proteins nach Anspruch 7 und einen physiologisch verträglichen Träger.

13. Zusammensetzung, geeignet zur Verabreichung an einen Patienten, umfassend eine therapeutisch wirksame Menge von Antikörpern gegen ein Protein nach Anspruch 7, die fähig sind dessen physiologische Aktivität zu blockieren, und einen verträglichen Träger.

**Revendications**

1. Isolat d'acide nucléique comprenant les nucléotides 1-564 de la figure 1.

2. Acide nucléique ayant plus de 10 pb, qui est capable de s'hybrider dans des conditions stringentes avec l'acide nucléique de la revendication 1 et codant pour une protéine ayant la capacité de moduler la croissance ou la différenciation des globules sanguins ou de leurs cellules précurseurs, ou ayant la capacité d'avoir une réaction croisée avec un anticorps dirigé contre ladite protéine modulatrice, les conditions stringentes étant définies comme un lavage en présence de NaCl 0,15 M / citrate de sodium 0,015 M / 0,1 % de NaDoSO$_4$ à 50°C, ou bien en présence de formamide à 50 % (vol/vol) avec 0,1 % de sérum-albumine bovine / 0,1 % de Ficoll / 0,1 % de polyvinylpyrrolidone / tampon phosphate de sodium 50 mM à pH 6,5 avec du NaCl 750 mM, du citrate de sodium 75 mM et une température de 42°C pour l'hybridation.

3. Vecteur réplicable comprenant un acide nucléique selon la revendication 1 ou 2.

4. Cellule hôte transformée avec un acide nucléique selon la revendication 1 ou 2.

5. Cellule hôte selon la revendication 4, qui est une cellule de mammifère.

6. Méthode de diagnostic de la leucémie, comprenant l'analyse d'une cellule leucémique pour rechercher une transcription ou une traduction de l'acide nucléique de la revendication 1 ou 2, ou pour déceler des modifications de structure de cet acide nucléique, comme des délétions, des réarrangements ou une amplification.

7. Protéine comprenant une séquence d'aminoacides pour laquelle code la séquence de l'acide nucléique de la revendication 1 ou 2.

8. Composition comprenant une protéine exempte de cellules selon la revendication 7.

9. Anticorps capable de fixer la protéine de la revendication 7.

10. Protéine selon la revendication 7, marquée avec un reste décelable.

11. Protéine selon la revendication 10, dans laquelle le reste est un isotope radioactif ou une enzyme.

12. Composition convenant à l'administration à un patient, comprenant une quantité thérapeutiquement efficace d'une protéine selon la revendication 7 et un support physiologiquement acceptable.

13. Composition convenant à l'administration à un patient, comprenant une quantité thérapeutiquement efficace d'anticorps dirigés contre une protéine selon la revendication 7, capables de bloquer son activité physiologique, et un support acceptable.

```
          10        20        30        40        50        60
ATTCGCGGCCCCTACAGTCGCCGCCGCTCCCCCAGCTACAGCCGCCACAGCTCCTACGAG
IleArgGlyProTyrSerArgArgArgSerProSerTyrSerArgHisSerSerTyrGlu
 PheAlaAlaProThrValAlaAlaAlaProProAlaThrAlaAlaThrAlaProThrSe
  SerArgProLeuGlnSerProProLeuProGlnLeuGlnProProGlnLeuLeuArgA


          70        80        90       100       110       120
CGGGGCGGCGACGTGTCCCCTAGTCCCTACAGCAGCAGCAGCTGGCGCCGCTCTCGCAGT
ArgGlyGlyAspValSerProSerProTyrSerSerSerSerTrpArgArgSerArgSer
 rGlyAlaAlaThrCysProLeuValProThrAlaAlaAlaAlaGlyAlaAlaLeuAlaVa
  laGlyArgArgArgValProEndSerLeuGlnGlnGlnGlnLeuAlaProLeuSerGlnS


         130       140       150       160       170       180
CCCTACAGCCCTGTGCTCAGACGGTCTGGAAAATCCCGAAGCAGAAGCCCGTATTCATCT
ProTyrSerProValLeuArgArgSerGlyLysSerArgSerArgSerProTyrSerSer
 lProThrAlaLeuCysSerAspGlyLeuGluAsnProGluAlaGluAlaArgIleHisLe
  erLeuGlnProCysAlaGlnThrValTrpLysIleProLysGlnLysProValPheIleE


         190       200       210       220       230       240
AGGCATTCAAGATCTCGTAGCAGGCACAGATTGTCTAGATCCAGAAGTCGTCATTCTAGT
ArgHisSerArgSerArgSerArgHisArgLeuSerArgSerArgSerArgHisSerSer
 uGlyIleGlnAspLeuValAlaGlyThrAspCysLeuAspProGluValValIleLeuVa
  ndAlaPheLysIleSerEndGlnAlaGlnIleValEndIleGlnLysSerSerPheEndT


         250       260       270       280       290       300
ATTTCTCCTAGCACACTAACTCTGAAGAGTAGCCTGGCAGCTGAATTGAACAAGAATAAA
IleSerProSerThrLeuThrLeuLysSerSerLeuAlaAlaGluLeuAsnLysAsnLys
 lPheLeuLeuAlaHisSerEndLeuEndArgValAlaTrpGlnLeuAsnEndThrArgIleLy
  yrPheSerEndHisThrAsnSerGluGluEndProGlySerEndIleGluGlnGluEndL


         310       320       330       340       350       360
AAAGCACGAGCAGCAGAGGCAGCAAGAGCCGCAGAAGCAGCGAAAGCTGCAGAAGCAACT
LysAlaArgAlaAlaGluAlaAlaArgAlaAlaGluAlaAlaLysAlaAlaGluAlaThr
 sLysHisGluGlnGlnArgGlnGlnGluProGlnLysGlnArgLysLeuGlnLysGlnLe
  ySerThrSerSerArgGlySerLysSerArgArgSerSerGluSerCysArgSerAsnE


         370       380       390       400       410       420
AAGGCTGCTGAGGCTGCTGCCAAGGCTGCAAAAGCTTCAAACACTTCTACACCTACCAAG
LysAlaAlaGluAlaAlaAlaLysAlaAlaLysAlaSerAsnThrSerThrProThrLys
 uArgLeuLeuArgLeuLeuProArgLeuGlnLysLeuGlnThrLeuLeuHisLeuProAr
  ndGlyCysEndGlyCysCysGlnGlyCysLysSerPheLysHisPheTyrThrTyrGlnG


         430       440       450       460       470       480
GGGAACACGGAAACTAGTGCCAGTGCATCACAAACAAACCATGTGAAGGATGTGAAGAAA
GlyAsnThrGluThrSerAlaSerAlaSerGlnThrAsnHisValLysAspValLysLys
 gGlyThrArgLysLeuValProValHisHisLysGlnThrMetEndArgMetEndArgLy
  lyGluHisGlyAsnEndCysGlnCysIleThrAsnLysProCysGluGlyCysGluGluA


         490       500       510       520       530       540
ATTAAAAATTGAACATGCACCTTCTCCCTCAAGTGGTGGAACTTTAAAAAAATGACAAAGCA
IleLysIleGluHisAlaProSerProSerSerGlyGlyThrLeuLysAsnAspLysAla
 sLeuLysLeuAsnMetHisLeuLeuProGlnValValGluLeuEndLysMetThrLysGl
  snEndAsnEndThrCysThrPheSerLeuLysTrpTrpAsnPheLysLysEndGlnSerL
```

Fig. 1a

```
            550         560      ↓ 570        580         590         600
AAAACAAAAGCCTACCTCTTCAGGTAACGAAGGTGGAAAATAATTTGATTGTAGATAAAG
LysThrLysAlaTyrLeuPheArgEndArgArgTrpLysIleIleeEndLeuEndIleLys
nLysGlnLysProThrSerSerGlyAsnGluGlyGlyLysEndPheAspCysArgEndSe
ysAsnLysSerLeuProLeuGlnValThrLysValGluAsnAsnLeuIleValAspLysA


            610         620         630         640         650         660
CCACCAAGAAAGCAGTCATAGTTGGAAAGGAGAGTAAATCTGCTGCTACAAAGGAGGAAT
ProProArgLysGlnSerEndLeuGluArgArgValAsnLeuLeuLeuGlnArgArgAsn
rHisGlnGluSerSerHisSerTrpLysGlyGluEndIleCysCysTyrLysGlyGlyIl
laThrLysLysAlaValIleValGlyLysGluSerLysSerAlaAlaThrLysGluGluS


            670         680         690         700         710         720
CAGTATCTCTTAAAGAGAAAACCAAACCACTTACACCAAGCATAGGAGCCAAGGAGAAGG
GlnTyrLeuLeuLysArgLysProAsnHisLeuHisGlnAlaEndGluProArgArgArg
eSerIleSerEndArgGluAsnGlnThrThrTyrThrLysHisArgSerGlnGlyGluGl
erValSerLeuLysGluLysThrLysProLeuThrProSerIleGlyAlaLysGluLysG


            730         740         750         760         770         780
AGCAACATGTAGCTTTAGTCACCTCTACATTACCACCGTTACCTTTGCCTCCCATGCTGC
SerAsnMetEndLeuEndSerProLeuHisTyrHisArgTyrLeuCysLeuProCysCys
yAlaThrCysSerPheSerHisLeuTyrIleThrThrValThrPheAlaSerHisAlaAl
luGlnHisValAlaLeuValThrSerThrLeuProProLeuProLeuProProMetLeuP


            790         800         810         820         830         840
CTGAAGATAAAGAAGCTGATAGCTTACGAGGAAATATTTCAGTAAAAGCAGTTAAAAAAG
LeuLysIleLysLysLeuIleAlaTyrGluGluIlePheGlnEndLysGlnLeuLysLys
aEndArgEndArgSerEndEndLeuThrArgLysTyrPheSerLysSerSerEndLysAr
roGluAspLysGluAlaAspSerLeuArgGlyAsnIleSerValLysAlaValLysLysG


            850         860         870         880         890         900
AAGTAGAAAAGAAACTCCGATGTCTTCTTGCTGATTTACCGCTGCCCCCCTGAGCTACCAG
LysEndLysArgAsnSerAspValPheLeuLeuIleTyrArgCysProLeuSerTyrGln
gSerArgLysGluThrProMetSerSerCysEndPheThrAlaAlaProEndAlaThrAr
luValGluLysLysLeuArgCysLeuLeuAlaAspLeuProLeuProProGluLeuProG


            910         920         930         940         950         960
GAGGAGATGATCTTTCAAAGAGTCCAGAGGAAAAGAAAACAACAACACAGTTACATAGTA
GluGluMetIlePheGlnArgValGlnArgLysArgLysGlnGlnHisSerTyrIleVal
gArgArgEndSerPheLysGluSerArgGlyLysGluAsnAsnAsnThrValThrEndEn
lyGlyAspAspLeuSerLysSerProGluGluLysLysThrThrThrGlnLeuHisSerL


            970         980         990        1000        1010        1020
AAAGGAGGCCTAAAATATGTGGGCCTCGCTATGGTGAAACAAAGAAAAAGATATTGACTG
LysGlyGlyLeuLysTyrValGlyLeuAlaMetValLysGlnArgLysArgTyrEndLeu
dLysGluAlaEndAsnMetTrpAlaSerLeuTrpEndAsnLysGluLysAspIleAspTr
ysArgArgProLysIleCysGlyProArgTyrGlyGluThrLysLysLysIleLeuThrG


           1030        1040        1050        1060        1070        1080
GGGAAAACGCTGCGTGGATAAATTTGATATCATCGGAATTATTGGAGAAGGTACTTACGG
GlyLysThrLeuArgGlyEndIleEndTyrHisArgAsnTyrTrpArgArgTyrLeuArg
pGlyLysArgCysValAspLysPheAspIleIleGlyIleIleGlyGluGlyThrTyrGl
lyGluAsnAlaAlaTrpIleAsnLeuIleSerSerGluLeuLeuGluLysValLeuThrA
```

Fig 1b.

21

EP 0 354 989 B1

```
        1090      1100      1110      1120      1130      1140
ACAAGTTTACAAAGCCAGGGATAAGACACTGGAGAAATGGTAGCCCTTAAAAAAAGTACGT
ThrSerLeuGlnSerGlnGlyyEndAspThrGlyyGluMetValAlaLeuLysLysValArg
yGlnValTyrLysAlaArgAspLysThrLeuGluLysTrpEndProEndLysLysTyrVa
spLysPheThrLysProGlyIleArgHisTrpArgAsnGlySerLeuLysLysLysSerThrS


        1150      1160      1170      1180      1190      1200
CTGGATAATGAAACGGAAGGCTTTCCAATTACAGCAATTCGAGAAATTAAAATTCTCCGG
LeuAspAsnGluThrGluGlyyPheProIleThrAlaIleArgGluIleLysIleLeuArg
lTrpIleMetLysArgLysAlaPheGlnLeuGlnGlnPheGluLysLeuLysPheSerGl
erGlyEndEndAsnGlyArgLeuSerAsnTyrSerAsnSerArgAsnEndAsnSerProA


        1210      1220      1230      1240      1250      1260
CAGCTTACCCATCAGAGTATTATCAATATGAAGGAAATAGTGACTGATAAAGAAGATTGC
GlnLeuThrHisGlnSerIleIleAsnMetLysGluIleValThrAspLysGluAspCys
ySerLeuProIleArgValLeuSerIleEndArgLysEndEndLeuIleLysLysIleAl
laAlaTyrProSerGluTyrTyrGlnTyrGluGlyAsnSerAspEndEndArgArgLeuL


        1270      1280      1290      1300      1310      1320
TTGGATTTCAAGAAGGACAAAGGTGCATCTATCTGTGCTGATATATGGACCATGATTTGA
LeuAspPheLysLysAspLysGlyAlaSerIleCysAlaAspIleTrpThrMetIleEnd
aTrpIleSerArgArgThrLysValHisLeuSerValLeuIleTyrGlyProEndPheAs
euGlyPheGlnGluGlyGlnArgCysIleTyrLeuCysEndTyrMetAspHisAspLeuM


        1330      1340      1350      1360      1370      1380
TGGACTACTGGAATCAGGCTTGGTCATTTTATGAAAATCACATAAAGTCATTTATGAGAC
TrpThrThrGlyyIleArgLeuGlyHisPheMetLysIleThrEndSerHisLeuEndAsp
pGlyLeuLeuGluSerGlyyLeuValIleLeuEndLysSerHisLysValIleTyrGluTh
etAspTyrTrpAsnGlnAlaTrpSerPheTyrGluAsnHisIleLysSerPheMetArgG


        1390      1400      1410      1420      1430      1440
AGCTCATGGAGGGTCTGGATTATTGTCATAAGAAGAACTTTTTGCATAGAGATATTAAAT
SerSerTrpArgValTrpIleIleValIleArgArgThrPheCysIleGluIleLeuAsn
rAlaHisGlyGlySerGlyLeuLeuSerEndGluGluLeuPheAlaEndArgTyrEndMe
lnLeuMetGluGlyLeuAspTyrCysHisLysLysAsnPheLeuHisArgAspIleLysC


        1450      1460      1470      1480      1490      1500
GTTCCAATATCCTTCTAAATAATAGAGGGCAGATAAAACTTGCAGACTTTGGACTTGCTC
ValProIleSerPheEndIleIleGluGlyArgEndAsnLeuGlnThrLeuAspLeuLeu
tPheGlnTyrProSerLysEndEndArgAlaAspLysThrCysArgLeuTrpThrCysSe
ySerAsnIleLeuLeuAsnAsnArgGlyGlnIleLysLeuAlaAspPheGlyLeuAlaA


        1510      1520      1530      1540      1550      1560
GATTGTATAGCTCAGAAGAAAGTCGGCCGTATACTAACAAGGTAATTACTTTATGGTACC
AspCysIleAlaGlnLysLysValGlyArgIleLeuThrArgEndLeuLeuTyrGlyThr
rIleValEndLeuArgArgLysSerAlaValTyrEndGlnGlyAsnTyrPheMetValPr
rgLeuTyrSerSerGluGluSerArgProTyrThrAsnLysValIleThrLeuTrpTyrA


        1570      1580      1590      1600      1610      1620
GTCCACCTGAACTGCTACTGGGAGAAGAACGATACACACCAGCCATTGATGTATGGAGCT
ValHisLeuAsnCysTyrTrpGluLysAsnAspThrHisGlnProLeuMetTyrGlyAla
aSerThrEndThrAlaThrGlyArgArgThrIleHisThrSerHisEndCysMetGluLe
rgProProGluLeuLeuLeuGlyGluGluArgTyrThrProAlaIleAspValTrpSerC
```

Fig. 1c

```
          1630        1640        1650        1660        1570        1680
GTGGCTGTATCCTTGGCGAACTCTTCACTAAAAAACCTATATTTCAAGCAAATCAGGAAC
ValAlaValSerLeuAlaAsnSerSerLeuLysAsnLeuTyrPheLysGlnIleArgAsn
 uTrpLeuTyrProTrpArgThrLeuHisEndLysThrTyrIleSerSerLysSerGlyTh
  ysGlyCysIleLeuGlyGluLeuPheThrLysLysProIlePheGlnAlaAsnGlnGluL


          1690        1700        1710        1720        1730        1740
TTGCACAACTAGAATTAATAAGCCGAATATGTGGGAGTCCATGTCCTGCAGTGTGGCCTG
LeuHisAsnEndAsnEndEndAlaGluTyrValGlyValHisValLeuGlnCysGlyLeu
 rCysThrThrArgIleAsnLysProAsnMetTrpGluSerMetSerCysSerValAlaEn
  euAlaGlnLeuGluLeuIleSerArgIleCysGlySerProCysProAlaValTrpProA


          1750        1760        1770        1780        1790        1800
ATGTAATCAAACTACCATATTTCAACACCATGAAACCAAAGAAGCAATATCGTCGAAAGT
MetEndSerAsnTyrHisIleSerThrProEndAsnGlnArgSerAsnIleValGluSer
 dCysAsnGlnThrThrIlePheGlnHisHisGluThrLysGluAlaIleSerSerLysVa
  spValIleLysLeuProTyrPheAsnThrMetLysProLysLysGlnTyrArgArgLysL


          1810        1820        1830        1840        1850        1860
TAAGAGAAGAATTGTTTTTATTCCTGCAGCTGCGCTAGACTTATTTGATTACATGCTTGC
EndGluLysAsnCysPheTyrSerCysSerCysAlaArgLeuIleEndLeuHisAlaCys
 lLysArgArgIleValPheIleProAlaAlaAlaLeuAspLeuPheAspTyrMetLeuAl
  euArgGluGluLeuPheLeuPheLeuGlnLeuArgEndThrTyrLeuIleThrCysLeuP


          1870        1880        1890        1900        1910        1920
CTTGGATCCTAGTAAGCGCTGCACTGCTGAACAGGCTCTTCAGTGCGAGTTCCTCCGAGA
LeuGlySerEndEndAlaLeuHisCysEndThrGlySerSerValArgValProProArg
 aLeuAspProSerLysArgCysThrAlaGluGlnAlaLeuGlnCysGluPheLeuArgAs
  roTrpIleLeuValSerAlaAlaLeuLeuAsnArgLeuPheSerAlaSerSerSerGluM


          1930        1940        1950        1960        1970        1980
TGTGGAACCCTCAAAAAATGCCTCCACCAGATCTCCCTTTATGGCAAGATTGTCATGAGTT
CysGlyThrLeuLysAsnAlaSerThrArgSerProPheMetAlaArgLeuSerEndVal
 pValGluProSerLysMetProProProAspLeuProLeuTrpGlnAspCysHisGluLe
  etTrpAsnProGlnLysCysLeuHisGlnIleSerLeuTyrGlyLysIleValMetSerT


          1990        2000        2010        2020        2030        2040
ATGGAGTAAAAAGCGAAGAAGACAGAAGCAGATGGGCATGATGACTGATGTTTCCACAAT
MetGluEndLysAlaLysLysThrGluAlaAspGlyHisAspAspEndCysPheHisAsn
 uTrpSerLysLysArgArgArgGlnLysGlnMetGlyMetMetThrAspValSerThrIl
  yrGlyValLysSerGluGluAspArgSerArgTrpAlaEndEndLeuMetPheProGlnL


          2050        2060        2070        2080        2090        2100
TAAAGCCCCCAGGAAGGACTTGTCTCTGGGCTTGGATGACAGCAGAACCAACACACCCCA
EndSerProGlnGluGlyLeuValSerGlyLeuGlyEndGlnGlnAsnGlnHisThrPro
 eLysAlaProArgLysAspLeuSerLeuGlyLeuAspAspSerArgThrAsnThrProGl
  euLysProProGlyArgThrCysLeuTrpAlaTrpMetThrAlaGluProThrHisProA


          2110        2120        2130        2140        2150        2160
GGGTGTGCTGCCATCTTCACAGCTGAAATCTCAGGGCACTCAAATGTGGCACCTGGTGAA
GlyCysAlaAlaIlePheThrAlaGluIleSerGlyHisSerAsnValAlaProGlyGlu
 nGlyValLeuProSerSerGlnLeuLysSerGlnGlyThrGlnMetTrpHisLeuValLy
  rgValCysCysHisLeuHisSerEndAsnLeuArgAlaLeuLysCysGlyThrTrpEndL
```

Fig 1d

```
       2170        2180        2190        2200        2210
AAACAGACAGATCCATCAACACCACAACAGGAGTCTTCGAAACCGTGGAGGAA
LysGlnThrAspProSerThrProGlnGlnGluSerSerLysProTrpArg
sAsnArgGlnIleHisGlnHisHisAsnArgSerLeuArgAsnArgGlyGly
ysThrAspArgSerIleAsnThrThrThrGlyValPheGluThrValGluGlu
```

Fig. 1e